# EUROPEAN PATENT SPECIFICATION

(11) **EP 1 633 428 B1**
(45) Date of publication and mention of the grant of the patent: **23.11.2011**
(21) Application number: 04735488.1
(22) Date of filing: 31.05.2004
(51) Int. Cl.: A61M 39/10, A61M 1/16, A61M 1/28

(54) **A JOINT FOR FLUID TRANSPORT LINES FOR MEDICAL USE**
KUPPLUNG ZUM FÖRDERN VON FLÜSSIGKEITEN FÜR MEDIZINISCHE ZWECKE
RACCORDS POUR CONDUITS DE FLUIDES À DES FINS MÉDICALES

(30) Priority: 04.06.2003 IT MO20030165
(43) Date of publication of application: 15.03.2006
(73) Proprietor: Gambro Lundia AB, 226 43 Lund (SE)
(72) Inventor: BARALDI, Vincenzo, 46026 Quistello (IT); DELNEVO, Annalisa, 40019 Sant'Agata Bolognese (IT); MARCHESI, Gianfranco, 41031 Camposanto (IT); LIGABUE, Andrea, 41030 San Prospero (IT); ZACCARELLI, Massimo, 41038 San Felice sul Panaro (IT)
(74) Representative: Ponzellini, Gianmarco
(86) International application number: PCT/IB2004/001777
(87) International publication number: WO 2004/108206

(56) References cited:
- EP-A- 0 542 140
- FR-A- 2 547 504
- GB-A- 1 033 971
- US-A- 3 580 983
- US-A- 4 215 384
- US-A- 5 127 907
- US-A1- 2003 036 719

## Description

### DESCRIPTION

### Background of the Invention.

The invention relates to a joint for fluid transport lines for medical use, to a fluid transport line comprising the joint, to an infusion device comprising the line, to a circuit for extracorporeal blood treatment comprising the line, to a machine for extracorporeal blood treatment which is operatively associable to the circuit, and to an apparatus for extracorporeal treatment of blood comprising the machine and the circuit.

Specifically, though not exclusively, the invention can be usefully applied in the field of intensive treatment of acute renal insufficiency.

In the prior art, renal insufficiency, both chronic and acute, is treated by extracorporeal dialytic treatment, in which blood is removed from the patient through a withdrawal line (arterial line) of an extracorporeal circuit, is sent to a first chamber (blood chamber) of a device for extracorporeal blood treatment (dialyzer or dialyzer filter, or artificial kidney), and is returned to the patient through a return line (venous line) of the extracorporeal circuit.

The treatment device comprises a second chamber (dialysis chamber) which is separated from the first by a semi-permeable membrane. The second chamber has an outlet, fluidly connected to a drainage line for a discharge fluid, and generally also has an inlet, fluidly connected to a supply line of a fresh dialysis fluid.

In some treatments, especially in intensive therapy for treatment of acute renal insufficiency, one or more infusion lines can be provided, in particular a first infusion line, for supply of a first infusion fluid into the blood withdrawal line upstream of the dialyzer filter (pre-infusion), and a second infusion line, for supply of a second infusion fluid into the blood return line, downstream of the dialyzer filter (post-infusion).

To set up the treatment, the extracorporeal circuit is associated to a dialysis machine, which comprises at least one blood pump, in general a peristaltic pump, which is predisposed on the withdrawal line and is for the circulation of the blood. Usually the machine also comprises various other pumps, also usually peristaltic, for the circulation of the various fluids which flow in the other fluid transport lines: a drainage pump for circulating the discharge fluid along the drainage line; a pump for circulation of the fresh dialysis fluid along the supply line to the second chamber of the dialyzer filter; and an infusion pump for each infusion line.

Normally, during the course of extracorporeal treatment, some of the patient's physiological parameters are monitored, in particular it is usual to perform the patient's ECG.

One of the problems encountered during a dialysis treatment, especially in cases of intensive therapy, is that the rotation of the peristaltic pumps, in particular the blood pump, causes disturbances (known as artefacts) in the ECG.

This interference problem in the ECG is found both in complex apparatus, such as a dialysis machine for intensive treatment, as well as in more simple apparatus, such as an infusion device comprising an infusion line with a peristaltic pump.

The alteration in the ECG recording can lead to an indistinguishable tracing, or can cause distortions that might be wrongly interpreted and confused with signs of cardiac anomalies.

### Summary of the Invention.

A main aim of the present invention is to provide a solution to the above-described problem existing in the prior art.

A further aim of the invention is to realize a fluid transport line that can be incorporable in a circuit for extracorporeal circulation of blood and/or medical fluids, thanks to which it is possible to eliminate ECG interference that can be traced to the operation of the machine associated to the circuit and which comprises means for circulation of the fluid in the circuit itself.

A further aim of the invention is to make available a machine for extracorporeal blood treatment, to which an extracorporeal circuit is operatively associable and which includes the above-cited fluid transport line, the functioning of which does not cause disturbances to the patient's ECG.

A further aim of the invention is to provide an infusion device, in which a medical infusion liquid is placed in circulation along an infusion line by a pump, thanks to which device it is possible to eliminate interferences which disturb the ECG and which are due to the operation of the pump.

An advantage of the invention is that it offers a simple and economical solution to the above-described problem of ECG artefacts caused by the operation of an apparatus for extracorporeal blood treatment.

It is known from document EP0542140 a tube arrangement for use in blood purifying processes, comprising tubular pieces with at least one electrode consisting of electrically conducting plastic for the determination of parameters of the liquids flowing through the tube. The annular tubular pieces can be made alternately of conducting and insulating plastics and be bonded to one another. It is further known from document US3580983 a conductive line tubing with an elongated plastic tube and an elongated electrically conductive plastic filament secured to the tube, the plastic filament being made of flexible electrically conductive plastic material. It is further known from document US4215384 a hose construction having an inside surface defining a passage for conveying fluid under pressure and an electrical conductor disposed along the hose construction and having a pair of opposed terminal ends adapted to be placed in communication with the inside passage for dissipating static electricity caused by fluid flow through the passage. It is further known from document GB1033971 a connecting tube for catheters comprising an inner wall of latex or synthetic material having embedded in it a helical wire and having two reinforced end portions connected to the tube by coating the length of the tube with a latex or synthetic material solution which is made electrically conductive by mixing in it a conducting material such as carbon black. It is further known from document FR2547504 a device intended to suppress pruritus suffered by patients undergoing haemodialysis, the device consisting of two identical elements which join on the blood circuit of the haemodialyser generator and consisting of a tubing including a frayed aluminium foil electrode and an electrode made of steel, silver or another metal. Each electrode is connected to earth by an electrical circuit insulated from any other electrical circuit. The steel electrode captures molecules of oxalates and phosphates by electrostatic attachment and allows the frayed aluminium foil electrode to de-electrify the various blood molecules. It is further known from document US5127907 a system for eliminating or reducing static electricity in infusion pumping systems which may cause artifacts in electrocardiograms, by forming parts in relative motion of a similar material to reduce generation of static electric charges. It is further known from document US2003/036719 a device for monitoring the access to the cardiovascular system of a patient undergoing an extracorporeal treatment of blood, in which the device comprises a voltage generator for generating a potential difference between a part of the machine and a first point of a venous branch of the extracorporeal circuit, connecting the patient to the treatment device; a detector for detecting the value of a quantity that correlates with the electric current along at least one section of the venous branch between the first point and a venous needle fitted at the end of the venous branch and inserted in the vascular system of the patient, and calculating means for comparing the detected value with a reference range.

A further advantage is that the invention realizes an apparatus for extracorporeal blood treatment which eliminates ECG artefacts and which at the same time responds to the necessary requisites of electrical insulation, thus eliminating any risks involving the patient's well-being.

A further advantage of the invention is that it provides a solution which does not lead to any problems relating to bio-compatibility.

A still further advantage of the invention is that it provides a fluid transport line, simple and economical to manufacture, which is easily produced using known production processes.

These aims and advantages and more besides are all attained by the present invention, as it is characterized in one or more of the appended claims.

Further characteristics and advantages of the invention will better emerge from the detailed description of at least one preferred but non-exclusive embodiment of the invention, made herein below with reference to the accompanying figures of the drawings, which are given by way of example and which are non-limiting.

### Brief Description of the Drawings.

The description will be made with reference to the accompanying figures of the drawings, provided by way of non-limiting example, and in which:
- figure 1 is a diagram of a hydraulic circuit usable in a machine for intensive treatment according to the invention;
- figure 2 is a longitudinal section of a joint for a fluid transport line realized according to the invention;

- figure 3 shows the joint of figure 2 applied to a fluid transport line;
- figure 4 is a second embodiment of a joint according to the present invention;
- figure 5 is the joint of figure 4 applied to a fluid transport line;
- figure 6 is a support element which is applicable to a front surface of a dialysis machine, and is provided for removable fastening of the joint of figures 2 or 4;
- figure 7 shows the support element of figure 6 applied to the front surface of a dialysis machine;
- figure 8 shows section VIII-VIII of figure 7;
- figure 9 is a block diagram of the grounding of an extracorporeal circuit according to the present invention;
- figure 10 is a more detailed version of the electrical diagram of figure 9;
- figure 11 is a recording of an electrocardiograph applied to a dialysis apparatus during laboratory tests, where the invention is not applied;
- figure 12 is an ECG recording applied to the same apparatus as in figure 11, where the invention is applied;
- figure 13 compares two ECG recordings, taken during laboratory tests; in the first the conductive joint is not earthed, while in the second it is grounded with the interpositioning of a suitable safety grounding resistance;
- figure 14 is a diagram of an infusion device made according to the present invention.

### Detailed Description.

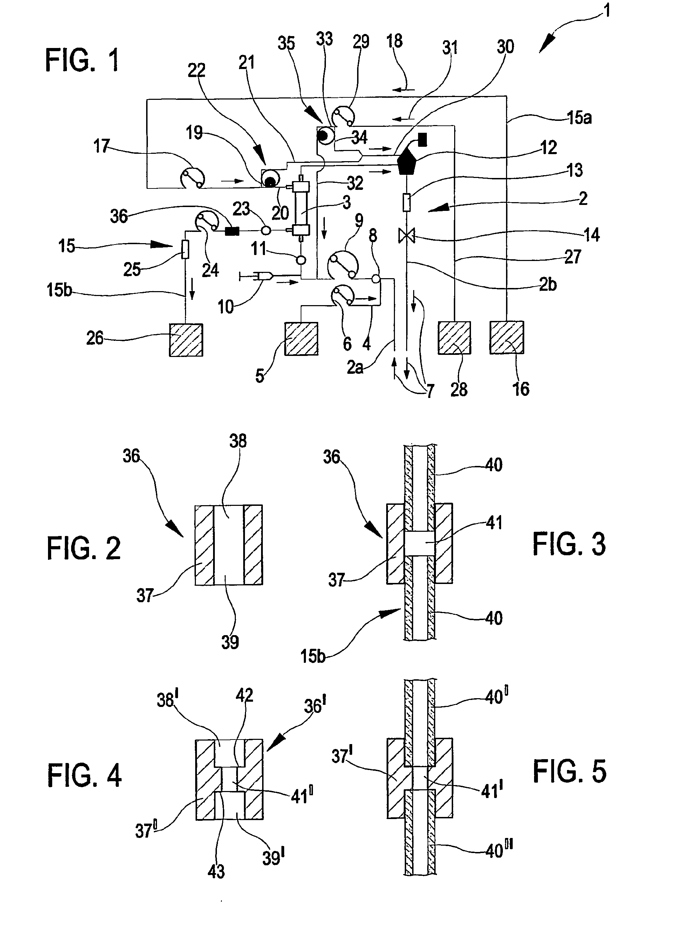

With reference to figure 1, the number 1 denotes an apparatus for extracorporeal treatment of blood, in particular a dialysis machine for intensive treatment.

A blood circuit 2 removes the blood from a patient, through a vascular access of known type and not illustrated, and, via at least one withdrawal line (inlet line or arterial line) 2a transports the blood, for example with continuous flow, to a blood treatment device 3 (or filtration unit, or dialyzer filter, or artificial kidney).

The blood crosses a first chamber (or blood chamber) of the blood treatment device 3 and, via a return line (or outlet line, or venous line) 2b, the treated blood is returned to the internal vascular system of the patient.

The withdrawal line 2a is connected, immediately downstream of the blood withdrawal zone, to an auxiliary, pre-infusion line 4.

A source of secondary fluid 5 (for example a container or bag), supplies the pre-infusion line 4. The apparatus comprises means for moving the fluid, in the illustrated example constituted by an auxiliary pre-infusion pump 6 (for example a peristaltic pump), which means for moving the fluid control the flow of secondary fluid injected directly into the blood via the pre-infusion line 4.

The source of secondary fluid 5 can supply a suitable biological fluid to effect a pre-infusion, but can also supply an anti-coagulant.

The blood flows, in a blood circulation direction 7, from the withdrawal line 2a towards the filtration unit, and from the filtration unit flow via the return line 2b back to the patient.

A blood pressure sensor 8 is predisposed immediately downstream of the auxiliary pre-infusion line 4.

The apparatus comprises means for moving fluid, i.e. in the particular case at least one blood pump 9 for control and management of the blood flow in the blood circuit 2. The blood pump 9 is generally peristaltic.

A device 10 for administering an anti-coagulant, for example a syringe containing appropriate doses of heparin, operates on the withdrawal line 2a downstream of the blood pump 9.

The blood passes a further pressure sensor 11 which monitors the correct flow into the blood circuit 2.

Then the blood enters the blood chamber of the treatment device 3, where, through a semi-permeable membrane, the desired substance, molecular and fluidic exchanges occur.

The treated blood, outletting from the treatment device 3, enters the return line 2b, crossing first a gas separator device (generally air) 12, predisposed to stop and expel any gassy substances or air bubbles present in the blood. The separator device 12 is operatively associated with a pressure sensor, of known type and not illustrated, for controlling the pressure in the return line 2b.

The treated blood outletting from the separator device 12 then crosses an air bubble sensor 13 which checks for absence of these dangerous formations internally of the treated blood.

Immediately downstream of the air bubble sensor 13 an intercept element 14 is located, the function of which is to block, during any alarms, the blood flow towards the patient.

Downstream of the intercept element 14 the treated blood is returned to the patient undergoing therapy.

A fluid circuit 15 is provided with at least one supply line 15a of a treatment fluid (fresh dialysis fluid), which enters a second chamber (dialysis chamber) of the treatment device 3, and a drainage line 15b outletting from the second chamber 3 of the device.

At least one source of treatment fluid 16 is connected to the supply line 15a of the fluid circuit 15 (the source of the treatment fluid 16 can be constituted, for example, by at least one bag containing a dialysis liquid).

The apparatus 1 comprises means for moving the fluid along the supply line 15a, including at least one supply pump 17 (in the illustrated embodiment a peristaltic pump), for controlling the flow of the treatment fluid coming from the source 16 and for defining a direction of circulation 18.

Downstream of the supply pump 17, along the circulation direction 18, there is a split 19 which divides the fluid circuit 15 into an injection branch 20 and an infusion branch 21. In particular the infusion branch 21 is connected to the return branch 2b of the blood circuit 2.

The infusion branch 21 enables a post-infusion directly into the blood circuit 2, using the treatment fluid coming from the source 16.

The injection branch 20 takes the treatment fluid directly to an inlet of the second chamber of the treatment device 3.

A selector switch 22 is predisposed in proximity of the split 19, and is for determining the percentage quantities of treatment fluid flow into the infusion branch 21 and into the injection branch 20. The selector switch 22, for example a cam switch or clamp switch, can assume at least a first operative configuration, in which the fluid is allowed to pass into the injection branch 20, and prevents passage into the infusion branch 21, and a second operative configuration, in which it allows passage into the infusion branch 21 and prevents passage into the injection branch 20. The selector switch 22 can modulate the quantities of fluid contemporaneously crossing one and the other branches 20 and 21, and can determine, including by programming, the changes in the quantities of fluids which flow in one branch or the other according to predetermined times and treatments.

The treatment fluid flowing in the injection branch 20 enters the second chamber (dialysis chamber) of the treatment device 3, which second chamber is separated from the first chamber (blood chamber) by the semi-permeable membrane which, as has already been mentioned, enables the correct substance exchanges between the blood and treatment fluid.

The fluid outletting from the second chamber of the treatment device 3, i.e. the discharge fluid, is transported by the drainage line 15b, also known as the effluent line.

A pressure sensor 23 is predisposed for controlling the functioning of the drainage line 15b.

Downstream of the pressure sensor 23 are located means for moving the fluid, for example a drainage pump 24, generally a peristaltic pump, able to control the flow in the drainage line 15b of the fluid circuit 15.

The discharge fluid crosses a blood leak detector 25 and is eliminated or directed into a container 26 for discharge fluid.

The apparatus comprises at least one further infusion line 27 which removes an infusion fluid from at least one auxiliary source 28 and, using means for moving fluid, usually a peristaltic infusion pump 29 which controls the flow, sends the fluid directly to the blood circuit 2 return line 2b. The infusion liquid can be introduced, as in the illustrated embodiment, directly into the gas separator device 12.

The infusion branch 21 of the fluid circuit 15 and the infusion line 27 are provided with a common end tract 30 for injection into the blood circuit 2. This end tract 30 is located downstream of the infusion pump 29 with respect to an infusion direction 14, and terminates directly in the separator device 12.

The infusion line 27 comprises at least one pre-infusion branch 32 connected to the withdrawal line 2a of the blood circuit 2. In more detail, downstream of the infusion pump 29 with respect to the infusion direction 31, there is a split 33 which divides the infusion line 27 into the pre-infusion branch 32 and a post-infusion branch 34.

The pre-infusion branch 32 transports the infusion fluid, taken from the container 28, towards the withdrawal line 2a of the blood circuit 2, downstream of the blood pump 9 with respect to the circulation direction 7.

The post-infusion line 34 is directly connected to the common end tract 30.

The infusion line 27 is provided with a selector switch 35, predisposed in proximity of the split 33, for determining the percentage quantity of the flow of liquid to be sent into the post-infusion branch 34 and the pre-infusion branch 32. The selector switch 35 can assume at least a first operative configuration, in which it allows passage of fluid into the pre-infusion branch 32 and prevents passage of fluid into the post-infusion branch 34, and at least a second operative configuration, in which it allows passage of fluid into the post-infusion branch 34 and prevents passage of fluid into the pre-infusion branch 32. The switch 35 can establish the percentage of fluid which must pass into each of the two branches 32 and 34, and can if necessary vary the times according to the treatments to be performed.

The apparatus 1 comprises a disposable part, usable in general for a single treatment, and a fixed part, which is used a number of times for various treatments on various patients. The fixed part is in effect the machine for extracorporeal blood treatment. The machine comprises, in general, a machine body which usually bears, on a front surface thereof, the various peristaltic pumps 6, 9, 17, 24 and 29, and also the various sensors, denoted by 8, 11, 13, 23 and 25, and the means for controlling flow, denoted by 14, 22 and 35, and an interfacing system with the operator, which generally comprises a display for entering and reading data.

The machine body also bears, internally, all of the electronic control circuitry, including a machine command unit.

The disposable part comprises the treatment device 3 and the blood circuit 2; in the illustrated embodiment, in which the apparatus serves to perform dialysis treatment of the intensive kind, the disposable part also comprises the dialysis circuit 15.

Substantially the machine integrates all of the instrumentation and apparatus destined to be used more than once, in various treatments, on one or more patients.

The disposable parts, destined to be used only once for each treatment to be performed on a patient, are borne on an integrated module, of known type and not illustrated, of the single-use type, applicable directly on the machine body.

The operation of the apparatus 1 includes a preliminary part, in which the disposable part is associated to the front surface of the machine body. During this phase the hydraulic circuit (blood circuit 2 and dialysis circuit 15) and the blood treatment circuit 3 are mounted on the machine in such a way that: the various peristaltic pumps engage the predisposed tracts of tubing (pump segments), which are generally U-shaped; all of the sensors are correctly engaged; and the containers of the various fluids are fluidly coupled to the respective fluid transport lines.

After the blood circuit 2 has been connected, in a known way, to a vascular access of a patient, the blood pump 9 is started up, which starts circulation of the blood in the circuit.

Thereafter, according to the type of treatment to be performed, the machine for extracorporeal blood treatment is automatically started up and controlled by the command unit.

The apparatus for extracorporeal blood treatment described above is able to perform treatments, in particular intensive treatments, each of which comprises one or more of the following treatments, with predeterminable sequences: pure ultrafiltration, haemofiltration, haemodialysis, haemodiafiltration, plasma exchange.

In figure 1, 36 denotes a joint for fluid transport lines for medical use, which is made according to the object of the invention. The joint 36 is predisposed along the drainage line 15b immediately downstream of the blood treatment device 3, that is, just after the outlet from the second chamber of the device 3 and before the drainage pump 24. In the illustrated embodiment, the joint 36 is located between the pressure sensor 23 and the drainage pump 24. This joint 36 will be described in more detail herein below.

The joint 36 is illustrated, in a first embodiment, in figure 2. In figure 3, the joint 36 is coupled to the drainage line 15b.

The joint 36 comprises a tubular body 37, substantially a sleeve-shape, having a cylindrical lateral external side and at two opposite ends two connecting zones 38 and 39, each of which has a cylindrical internal lateral surface for connecting.with an end zone of a usual tubular element 40 of a fluid transport line for medical use. The connection gives continuity to fluid passage.

Each tubular element 40 is a flexible elongate body, with elastically deformable walls, made of a dielectric plastic material, generally a thermoplastic resin, such as for example bio-compatible plasticized PVC.

The joint 36 is made in a single piece with a relatively small longitudinal extension having more rigid walls than the tubular elements 40.

In the illustrated example the joint 36 is made of a composite material including a mix of plastic material, generally a thermoplastic resin (for example the same material as the tubular elements 40, in this embodiment bio-compatible plasticized PVC), with at least one additive to give it electrical conductivity.

The combination of the above-mentioned additive with the thermoplastic resins, already dielectric, in suitable and known formulas, leads to obtaining a conductive material, though provided with relatively high electrical resistance.

The additive can be, for example, conductive carbon black, or another known product which, mixed with a thermoplastic resin, transforms the latter from being an insulator to being a conductor.

In the illustrated embodiment, the material, obtained from a mixture of a plastic material and a conductor additive, can be extruded by usual processes and apparatus used for PVC.

In more detail, in the illustrated embodiment, the selected material for the conductive joint 36 is CABELEC^{®} 3895, constituted by a compound including carbon black, plasticized PVC, stabilizer and lubricant.

The two connecting zones 38 and 39 of the joint are designed and structured to join the two tubular elements 40 solidly, one to another (even though axially distanced one from another), giving continuity to fluid passage. The two tubular elements 40, joined together by the joint 36, form a single conduit for the passage of a fluid.

The tubular body 37, made in a single piece, is produced by a plastic material pressing process.

The tubular body 37 is internally provided with at least one internal surface 41, destined to come into contact with the transported fluid, situated in an intermediate axial zone of the tubular body 37 comprised between the two end connecting zones 38 and 39.

The external surface of the tubular body 37 is destined to contact electrically with an element which is external of the fluid transport line, with the result that, via for example a grounded connection, the electrical currents present in the transported fluid transported in the fluid transport line can be dissipated. The external element, illustrated in figures from 6 to 8, will be better described herein below.

The tubular conductive joint 36, has a greater electrical conductivity than the tubular elements 40 which are reciprocally joined by the joint 36. The material of the tubular body 37 is, as has been mentioned, is based on a thermoplastic material, which in itself is dielectric, and which is made electrically conductive thanks to the addition, in the body of the plastic material, of carbon black or another suitable additive for obtaining electrical conductivity.

The joint 36 can therefore be considered an electrically conductive element, differently to the plastic tubular elements 40, which can be considered electric insulators.

The conductive joint 36 can be considered a high-resistance electrically-conductive element.

To achieve the desired aim, i.e. to considerably reduce or even eliminate disturbance of the ECG caused by electrostatic charges generated by the operation of peristaltic pumps, in particular the blood pump 9, the electrical impedance between the internal surface and the external surface of the tubular body 37 can vary within a range between 40 KΩ and 10 MΩ. As will be more fully explained herein below, a substantial elimination of electrocardiograph disturbances has been verified, with the ECG connected up to a patient being subjected to extracorporeal treatment, using, in the apparatus, a conductive joint 36 having an electrical impedance variable between 200 KΩ and 2 MΩ.

The material and conformation of the joint 36 simply and economically obtain a good, stable, resistant and well-sealed joint, between the jonit 36 and the tubular elements 40 which it joins. The joint union, permanently stable and unbreakable, can be obtained, during assembly, by a process of known type and already in use, for example, for solid connections by gluing of PVC tubes for medical products having corresponding plastic connectors. The procedure involves insertion of the end zones of the tubular elements 40 inside the connecting zones 38 and 39 of the joint 36, with a preliminary spreading on at least one of the coupling surfaces of a certain amount of a suitable glue, for example a cyclo-hexanone-based glue.

In a second embodiment, illustrated in figure 4, the conductive joint 36' is constituted by a tubular body 37', made in a single piece, which internally comprises at least one first axial stop element 42, operatively associated to an end zone of a first tubular element 40', for limiting an axial insertion of the first tubular element within the tubular body.

In the illustrated embodiment, the tubular body 37' internally comprises a second axial stop element 43, axially distanced from the first axial stop element 42, and operatively associated to an end zone of the second tubular element 40", for limiting an axial insertion of the second tubular element inside the tubular body 37', in an opposite direction with respect to the axial insertion of the first tubular element 40'.

The tubular body 37' has an intermediate zone 41' comprised axially between the two end connecting zones 38' and 39', the internal diameter of which is smaller than the internal diameter of the connecting zones. The intermediate zone 41', with a smaller diameter, offers an inwardly-directed annular recess, axially delimited by two abutments, which form the stop elements 42 and 43 which limit insertion of the end zones of the tubular elements 40' and 40". The elements 42. and 43 have the function of preventing total covering of the internal surface of the tubular body 37' by the tubular elements 40' and 40", so that a free intermediate zone 41' on the internal surface remains free, i.e. not covered by the end zones of the tubular elements 40' and 40", and in direct contact with the fluid which flows along the fluid transport line. This direct contact allows for dispersion to the outside of any electrostatic charges in the fluid.

The drainage line 15b of the apparatus 1 is an example of a fluid transport line, for medical use, made according to the invention.

The fluid transport line comprises at least a first part and a second part, both in contact with the transported fluid, in which the second part is made of a material having a greater electrical conductivity than the material the first part is made of. The second part of the line can comprise, as in the embodiment described herein, a conductive joint 36 or 36' like those first described, while the first part can comprise the tubular elements 40, 40', 40" described above.

The second part of line is also predisposed for galvanic connection to an element which is external of the line, as will be better explained herein below.

The conductive second part of the line exhibits at least one internal surface destined to contact with the transported fluid, and at least one external surface predisposed to be associated, in electrical contact, with a support element which is external of the line.

The first part is made of a thermoplastic material which is elastically deformable and dielectric, while the second part is made of a material composed of a mix of thermoplastic, dielectric material with the addition of at least one additive which gives the mixture a certain electrical conductivity.

The additive also has the property of giving greater rigidity to the mixture.

The second part of the electrically conductive line is situated, with reference to the fluid transport direction, upstream of a pump segment of the line. The pump segment is a tract of line, normally U-shaped and elastically deformable, which is operatively associated to a normally-peristaltic pump, for circulation of the transported fluid.

The second part of electrically-conductive line can be located, in other embodiments which are not illustrated, in any other point of the hydraulic circuit of figure 1, either in the fluid circuit 15 (or dialysis circuit) or in the blood circuit 2.

The location on the drainage line 15b, immediately downstream of the treatment device 3, has the advantage of ensuring an efficient electrical connection between the second part of line (the joint 36) and the blood circuit 2, without resorting to direct contact between the blood and the second part of electrically-conductive line.

It has been found that the treatment device 3, or dialyzer filter, does not constitute a barrier to electrical communication between the blood circuit 2 and the fluid circuit 15.

This advantage can be found also in hydraulic circuits which are different from the one illustrated in figure 1: in particular, the use of the second conductive part of line is effective also in simplified hydraulic circuits, such as for example a circuit for haemodiafiltration such as the one illustrated in figure 1, but lacking the branches 21 and 34, or a suitable circuit for effecting only haemofiltration, or a circuit suitable only for haemodialysis, or a circuit suitable only for pure ultrafiltration.

An apparatus for extracorporeal blood treatment, predisposed for cooperating with one of the above-cited hydraulic circuits, comprises at least one support element 44 predisposed to receive, with a mechanical engagement and in electrical contact, the above-mentioned second, electrically-conductive part of line (joint 36 or 36').

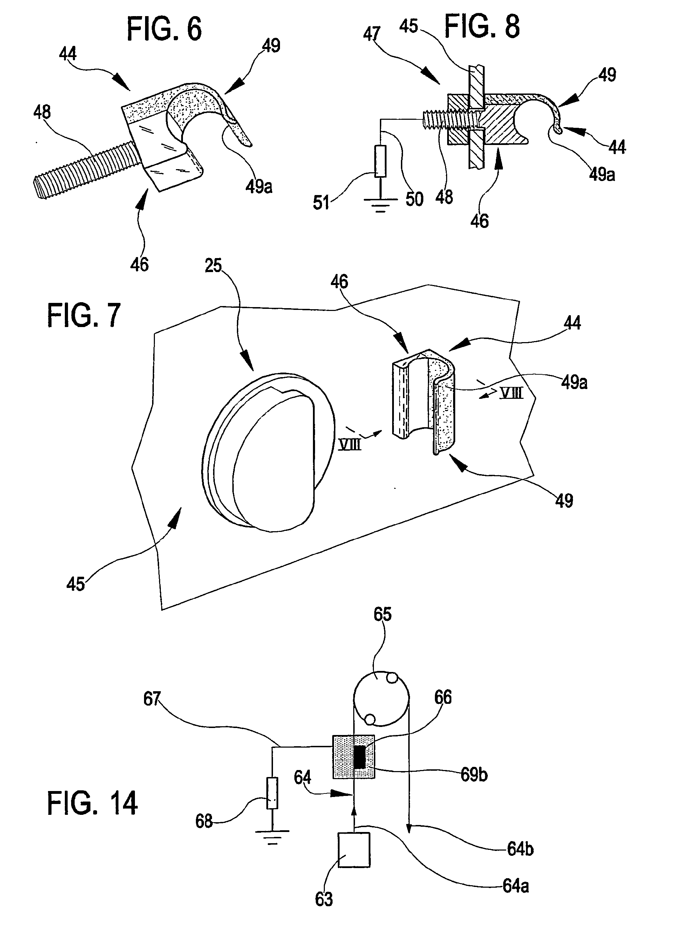

The support element 44 is solidly connected to a front panel 45 of the machine for extracorporeal blood treatment. An embodiment of this support element 44 is illustrated in figure 6, while figures 7 and 8 show the same support element 44 applied to the front panel 45 of the machine (in figure 7 the blood leak detector 25 can also be seen, located by the side of the support element 44).

The support element 44 comprises at least one electrically-conductive first part 46, made, for example, of metal, fixed to the front panel 45 of the machine by, for example, a screw connection 47. The conductive first part 46 can comprise a threaded stalk 48 for the screw connection with the front panel 45.

The support element 44 further comprises a second part 49, also dielectric and made of a plastic material, provided with a gripping organ 49a for removably fixing the fluid transport line to the conductive second part (the joint). The first and second parts 46 and 49 of the support element are solidly constrained one to another, for example by a screw connection (not illustrated).

The gripping organ 49a comprises, for example, a fastening, in the guise of an elastically deformable hook, which affords a seating in which the conductive joint 36 or 36' can be inserted and held tight in position. The joint 36 or 36' can be inserted and and removed manually from the seating.

The apparatus 1 further comprises a galvanic connection 50 which connects the support element 44 with an external mass, for external dissipation of any electrical charges present in the fluids, corporeal and/or medical, transported in the extracorporeal hydraulic circuit. The galvanic connection 50 terminates in the conductive first part 46 of the support element 44. In the illustrated embodiment the galvanic connection 50 is a true and proper earth for the joint 36, comprising at least one electrical earthing cable which connects the conductive first part of the support element, which is in contact with the above-mentioned conductive second part of line (joint 36 or 36'), with the machine body, which machine body is in turn normally provided with its own grounding.

The galvanic connection 50 also comprises at least one safety electrical impedance 51, of a predetermined entity, predisposed along the grounding cable between the support element 44 and the machine body. This safety impedance 51 guarantees the machine's electrical insulation, as required by the standards, together with the impedance value of the conductive joint 36 or 36'. The entity of the electrical impedance 51 can be, for example, above about 0.1 MΩ. It has been found that an efficient elimination of ECG artefacts (caused by the action of the peristaltic pumps) is also achieved with a safety impedance 51 of above about 1.0 MΩ.

Alternatively to the use of a single impedance 51 of about 1.0 MΩ, a plurality of electrical impedances, of predetermined entities (for example each not below about 2.0 MΩ), could be predisposed in parallel along the galvanic connection, with the aim of reducing the power dissipated.

The galvanic connection to earth can comprise, for example, an electronic board having: one or more impedances having predefined characteristics, at least a first contact for connecting to the conductive part 46 of the support element, and at least a second contact for connecting to the earthing cable.

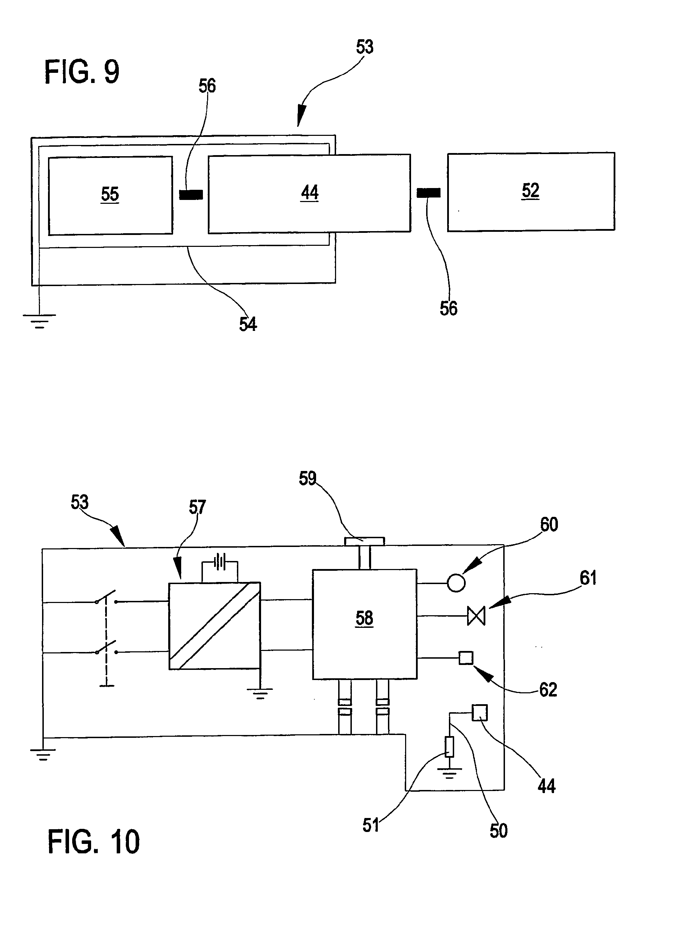

Figure 9 shows a block diagram of the electical earthing system of the hydraulic circuit of the apparatus 1. 52 denotes, in its entirety, the disposable part of the dialysis apparatus, which is provided with at least one conductive element in contact with at least one fluid which is transported along at least one tract of the hydraulic circuit of the apparatus. 53 denotes, in its entirety, the fixed part of the dialysis apparatus which comprises the support element 44, which, as mentioned, functions as a mechanical fastening and as an electrical contact for the conductive element of the disposable part. 54 denotes the machine body 54 of the machine, which is equipped with it own galvanic earthing connection 55, of known type. 56 denotes the electrical connections which connect up the various above-mentioned elements among themselves.

Figure 10 is a more detailed electrical diagram: 57 denotes the electric supply, 58 the machine command unit, 59 the operator interface display, 60 the entirety of the peristaltic pumps for circulation of the various fluids (corporeal and medical), 61 the entirety of the control organs for regulation of the various fluid transport lines (clamps, valves, selectors etc.), 62 the totality of the sensors (pressure, blood, air-bubble, any fluid-container weighing sensors there might be, and so on).

During the phase of readying the apparatus for operation, in which the disposable part is associated to the machine, the conductive joint 36 or 36' is pressure-fitted, simply and manually, in the seating constituted by the elastic fastening of the support element 44.

This simple operation makes possible the galvanic grounding connection of the discharge fluid circulating in the drainage line 15b of the dialysis fluid circuit.

Figures from 11 to 13 show the results of some laboratory tests performed to evaluate the effectiveness of the solution proposed in eliminating the ECG artefacts due to the rotation of the peristaltic pumps.

During the tests an apparatus comprising a machine for dialysis treatment was used, such as the one illustrated in figure 1, fitted with a disposable integrated module which includes both the blood circuit and the dialysis circuit, and also the dialyzer filter. The dialysis circuit used in the tests is the fluid circuit 15 of figure 1, minus branches 21 and 34.

A saline solution (9 g/l) was circulated in the blood circuit, taken from a container and returned to the same container; blood pump flow rate was fixed at 180 ml/min.

Four steel electrodes were immersed in the container, connected by a resistance to terminals L (47KΩ), R (380KΩ), F (47KΩ) and N (47KΩ) of an electrocardiograph. Terminal L was unbalanced by introducing, after the resistance, a 400pF condenser towards the ground. The slight unbalance of the impedance of electrode L transforms the common mode voltage produced by the rotation of the pump into a differential signal which is recorded by the ECG on I.

Before performing the test, the conductivity of the conductive joint 36 was measured. For this purpose, the joint was filled with saline solution (9 g/l) and the electric resistance between the external surface of the joint and the liquid inside was measured. The joint used in the tests had a resistance which varied between 200KΩ and 2 MQ.

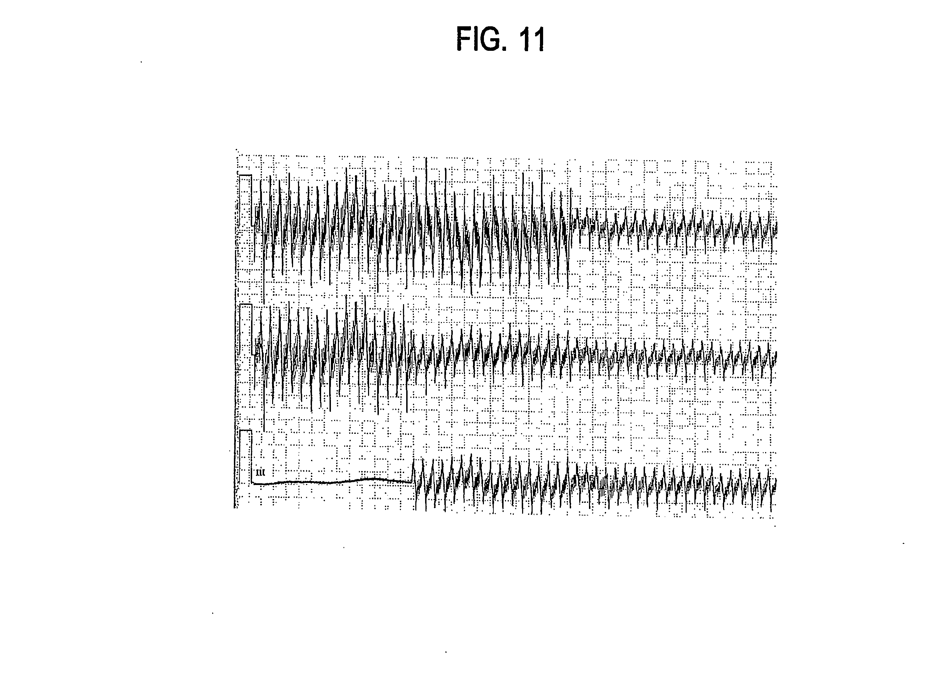

Figure 11, which shows the recording obtained with the conductive joint not grounded, evidences the disturbance produced by the pump rotation (paper speed 25 mm/sec, disturbance synchronous with movement of pump at about 6 c/s). There was disturbance on all cutouts with the exception of no. III, where disturbance is rejected and the impedances of the relative electrodes were exactly balanced. The automatic interpretation of the tracing gives abnormal ECG with atrial fibrillation, abnormal right axial deviation, unspecific intraventricular blockage.

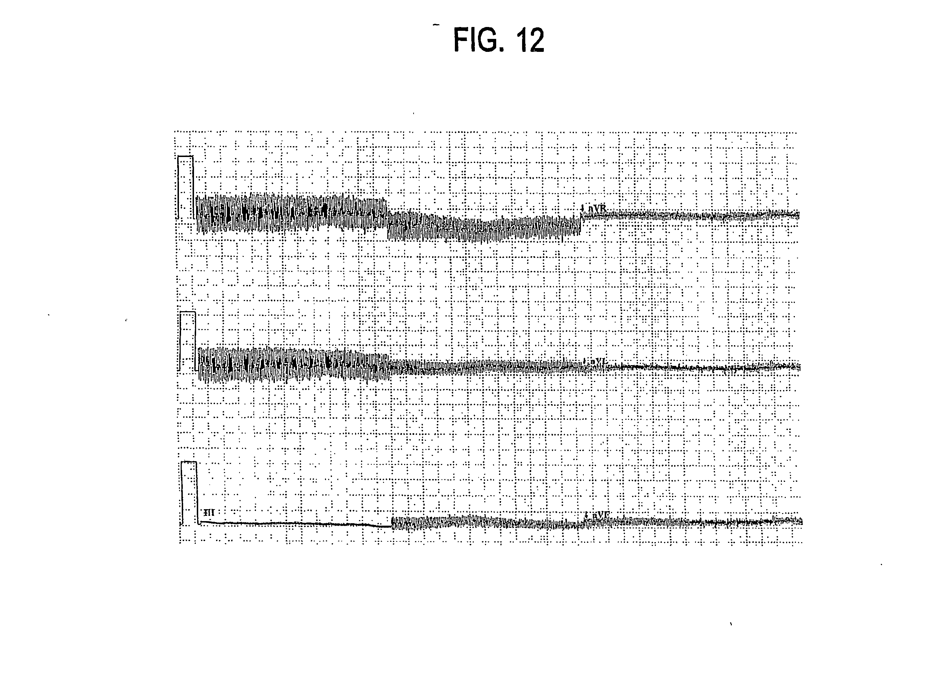

Figure 12 shows the ECG recording of the same test after the conductive joint, positioned on the effluent line immediately downstream of the dialyzer filter, has been galvanically connected to ground. The ground connection consists in connecting the joint by an electric cable to the machine body which in turn is grounded through the supply circuit.

By comparing the recording of figure 12 with that of figure 11, the ground connection considerably attenuates the disturbance produced by the movement of the blood pump. The automatic response provided by the ECG computer gives atypical ECG (as these were in vitro tests) and declares itself unable to give a complete interpretation.

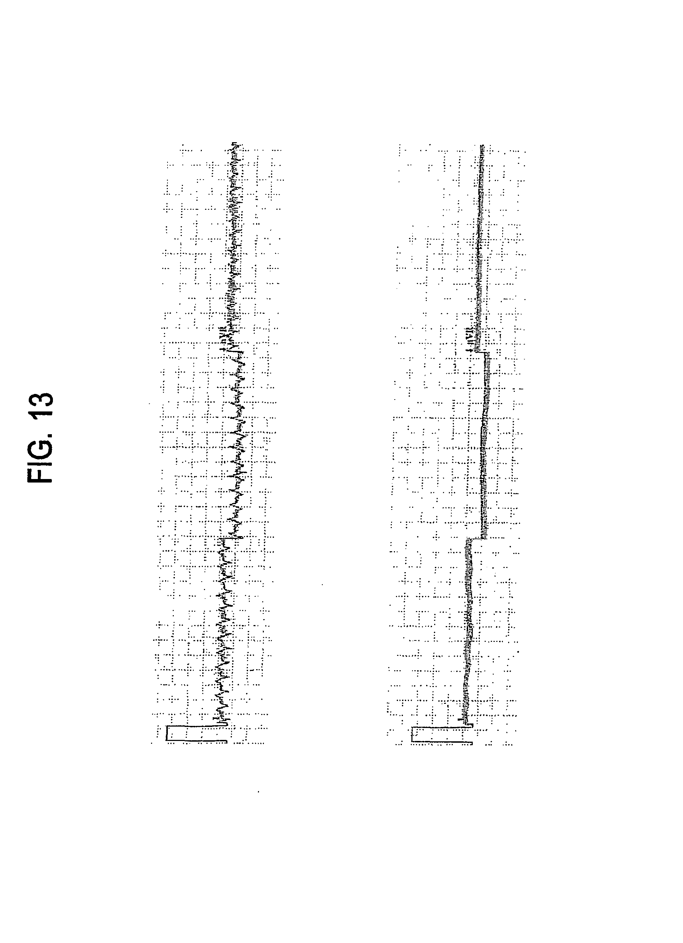

Figure 13 compares two test recordings. The top trace relates to a situation in which the conductive joint was not grounded: the automatic interpretation gives abnormal ECG with atrial flutter, epicardiac lesions, possibility of frontal infarct. The bottom trace relates to a situation in which the joint is grounded and in which, along the electric connecting cable between the joint and the body of the machine, a 1.2 MΩ resistance has been positioned. The automatic response describes an atypical ECG, but none of the negative interpretations given for the top tract.

The test result is a demonstration of the elimination of the ECG interference, even when a resistance is put in the ground connection which resistance is sufficient to conserve the requisite of electrical insulation of the machine.

The fluid transport line for medical use, comprising the conductive joint 36 or 36', as above described, can be used in the fields various typologies of medical apparatus where ECG interference is a problem. In this specific case the description relates to an apparatus for intensive treatment of acute renal insufficiency: it would be possible however to use the invention in other medical apparatus, such as for example dialysis apparatus for chronic renal insufficiency.

A further example is now described in more detail, of application of the conductive joint in an infusion device, with reference to figure 14.

The device comprises:
- a source 63 of an infusion liquid;
- an infusion line 64 having a first end, an inlet 64a, connected to the source 63 and a second end, an outlet 64b, which is placed in fluid communication, either directly or indirectly, with the vascular system of a patient;
- an infusion pump 65, for example a peristaltic pump, operatively associated to the infusion line 64 for circulating the infusion liquid;
- a conductive joint 66, made like the joint 36 or 36', predisposed along the infusion line 64 upstream of the pump 65;
- a galvanic connection 67 for connecting the conductive joint 66 with an external mass (for example the ground).

The device can further comprises a safety impedance 68, predisposed along the galvanic connection 67, having the function of guaranteeing that the electrical insulation for the patient undergoing the infusion treatment is in conformity with existing safety standards, and a mechanical fastening and electrical contact element, denoted by 69, to which the conductive joint 66 is applied, for example removably.

The infusion line 64 can be connected, directly to a vascular access of the patient, or indirectly to the patient, via an extracorporeal circuit.

In the case of an infusion device too, the material the conductive joint 66 is made of is a polymer which has been made conductive thanks to addition and mixing of carbon black or another known additive. As can be observed, in this case too the conductive joint 66 is located upstream of the peristaltic pump 65, with reference to the infusion fluid circulation direction.

The electrical contact element 69 destined to engage with the conductive joint 66, which can be once more, for example, an elastic fastening, can be solidly constrained to the pump body of the peristaltic pump 65.

The particular location, before the fluid circulation pump, of the conductive element guarantees reciprocal contact, constantly and in all operative situations, between the transported fluid and the conductive element.

In the illustrated embodiments the transport fluid which is galvanically connected to the outside is, in the first case (figure 1) the discharge fluid in the drainage line of a dialyzer filter, and in the second case (figure 14) the infusion fluid circulating along an infusion line, simple or cooperating with an extracorporeal blood circuit. Other transport fluids could, however, be galvanically connected to the outside, such as for example blood, circulating in the withdrawal line or the return line of an extracorporeal circuit, or fresh dialyzing fluid, circulating in the supply line of the dialysis chamber of a dialyzer filter, or the pre-infusion or post-infusion liquid of a dialysis circuit.

## Claims

1. A circuit for extracorporeal treatment of blood, comprising:
a blood treatment device (3) having a first chamber and a second chamber separated from the said first chamber by a semi-permeable membrane;
at least one blood withdrawal line (2a) for supplying blood, removed from a patient, to the said first chamber;
at least one blood return line (2b) for returning treated blood to the patient after the blood exits from the said first chamber;
at least one drainage line (15b) for draining a discharge fluid exiting from the said second chamber;
**characterized in that** the said drainage line (15b) comprises at least a first part (40; 40', 40") and a second part (36; 36'; 66), both of which are at least partially destined to contact the discharge fluid, the said second part (36; 36'; 66) being made of a material having a greater electrical conductivity than a material of which the said first part is made, the said second part of line also being predisposed for a galvanic connection with an element which is external of the drainage line, the said second part (36; 36'; 66) exhibiting at least one internal surface (41; 41') destined to contact the discharge fluid, and at least one external surface predisposed to be associated in electrical contact with a support element (44; 69), which support element (44; 69) is external of the drainage line, the said second part comprising a joint (36; 36'; 66) comprising a tubular body (37; 37') having at two opposite ends thereof two connecting zones (38, 39; 38', 39'), the said tubular body (37; 37') being made of an electrically conductive material; the said first part comprising two tubular elements (40; 40', 40") each of which has an end zone coupled to one of the said two connecting zones (38, 39; 38', 39').

2. The circuit of claim 1, **characterized in that** the said drainage line (15b) comprises a pump segment, designed to be operatively associated with a pump (24) for circulation of the drainage fluid along the said drainage line, and **in that** the said electrically-conductive second part (36; 36') is situated between an inlet end of the said drainage line and the said pump segment, the said inlet end being connected to an outlet of the said second chamber of the treatment device (3).

3. The circuit of claims 1 or 2, **characterized in that** the said tubular body (37; 37') is made of at least one plastic material containing at least one additive, which additive gives electric conductivity properties to the said tubular body (37; 37').

4. The circuit of claim 3, **characterized in that** the said additive comprises carbon and the said plastic material comprises PVC.

5. The circuit of any one of claims from 1 to 4, **characterized in that** the said joint has an internal surface (41; 41'), designed to contact the discharge fluid, and an external surface, designed to contact electrically with an element (44) which is external of the drainage line, in order to dissipate externally electric charges present in the discharge fluid, and **in that** an electrical resistance between the said internal surface and the said external surface being comprised between 40 KΩ and 10 MΩ.

6. The circuit of claim 5, **characterized in that** the said electrical resistance between the said internal surface and the said external surface is comprised between 200 MΩ and 2 MΩ.

7. The circuit of any one of claims from 1 to 6, **characterized in that** the said tubular body (37') comprises:
at least one first axial stop element (42; 43), operatively associated to an end zone of a first tubular element (40'; 40"), for limiting an axial insertion of the said first tubular element (40'; 40") inside the said tubular body; and
at least a second axial stop element (42; 43), axially distanced from the first axial stop element (42, 43), and operatively associated to an end zone of a second tubular element (40'; 40"), for limiting an axial insertion of the said second tubular element inside the said tubular body (37'); the axial insertion of the said second tubular element (40'; 40") in the tubular body (37') being done in an opposite direction to the axial insertion therein of the said first tubular element (40'; 40").

8. The circuit of any one of claims from 1 to 7, **characterized in that**:
the said tubular body (37, 37') has a free intermediate zone not used for connecting, which zone is axially comprised between the said connecting zones (38, 39; 38', 39') and destined for contact with the discharge fluid; and **in that**
the said intermediate zone (41') has an internal diameter which is smaller than an internal diameter of the said connecting zones (38', 39').

9. The circuit of any one of claims from 1 to 8, **characterized in that** the said end zones of the said tubular elements are inserted inside the said tubular body, which tubular body exhibits an axial intermediate zone (41; 41'), comprised between the said connecting zones (38, 39; 38', 39'), which is not occupied by the said end zones of the said tubular elements and which is in contact with the discharge fluid.

10. The circuit of any one of claims from 1 to 9, **characterized in that** the said tubular body (37; 37') is made in a single piece.

11. The circuit of any one of the preceding claims, **characterized in that** the material of the said first part of line is an elastically deformable plastic material, and **in that** the material of the said second part of line is an elastically-deformable plastic material, with an addition of at least one additive having properties making the material of the said second part of line electrically conductive.

12. The circuit of claim 11, **characterized in that** the said additive gives greater rigidity to the material of the said second part of line.

13. The circuit of any one of the preceding claims, **characterized by** further comprising one or more lines for circulation of a fluid selected from a group of lines, as follows:
at least one supply line (15a) for supplying an operative fluid to the said second chamber of the treatment device;
at least a first infusion line (4) for infusion of a substitution fluid to the said blood withdrawal line (2a);
at least a second infusion line (27), for infusion of a substitution fluid to the said blood return line (2b).

14. An apparatus for extracorporeal blood treatment comprising a circuit as in any one of claims from 1 to 13 and a machine for extracorporeal blood treatment, predisposed for use with a circuit made according to any one of claims from 1 to 13, the machine comprising:
at least one blood pump (9) for circulation of blood in the blood withdrawal line (2a);
at least one drainage pump (24) for circulation of discharge fluid in the drainage line (2b);
at least one support element (44) predisposed to receive, in electrical contact, the said electrically-conductive second part of line;
at least one galvanic connection (50, 51) which connects the said support element with an external mass, to dissipate electrical charges possibly present in the said circuit.

15. The apparatus of claim 14, **characterized in that** the said galvanic connection comprises a grounding.

16. The apparatus of claim 14 or 15, **characterized in that** the said galvanic connection comprises at least one electric cable (50) which connects the said support element (44) to a machine body which is in turn connected to ground.

17. The apparatus of any one of claims from 14 to 16, **characterized by** comprising at least one electrical safety impedance (51), of a predetermined entity and predisposed along the said galvanic connection.

18. The apparatus of claim 17, **characterized in that** the entity of the said electrical impedance (51) is greater than 0.1 MΩ.

19. The apparatus of claim 17 or 18, **characterized in that** the entity of the said electrical impedance (51) is equal to or greater than 1.0 MΩ.

20. The apparatus of any one of claims from 17 to 19, **characterized by** comprising a plurality of electrical impedances of predetermined entities, predisposed in parallel along the said galvanic connection.

21. The apparatus of any one of claims from 14 to 20, **characterized in that** the said support element (44) comprises at least a first part (46) made of an electrically conductive material, fixed to a bearing structure of the machine and at which the said galvanic connection (50) terminates, and a second part (49), made of a plastic material, provided with at least one organ (49a) for removable fastening of the said second electrically-conductive part of line thereto.

## Patentansprüche

1. Kreislauf für extrakorporale Blutbehandlung, umfassend:
eine Blutbehandlungsvorrichtung (3) mit einer ersten Kammer und einer zweiten Kammer, dievon der ersten Kammer durch eine semipermeable Membran getrennt ist;
mindestens eine Blutentnahmeleitung (2a) zur Zuführung von Blut, das von einem Patienten entnommen wurde, in die erste Kammer;
mindestens eine Blutrückgabeleitung (2b) zur Rückgabe behandelten Blutes zum Patienten nach dem das Blut aus der ersten Kammer austritt;
mindestens eine Abflussleitung (15b) zum Abfließenlasseneiner Ablaufflüssigkeit,
die aus der zweiten Kammer austritt;
**dadurch gekennzeichnet, dass** die Abflussleitung (15b) mindestens einen ersten Abschnitt (40; 40', 40") und einen zweiten Abschnitt (36; 36'; 66) umfasst, die beide zumindest teilweise dafür vorgesehen sind, mit der Ablaufflüssigkeit in Berührung zu kommen, wobei der zweite Abschnitt (36; 36', 66) aus einem Material mit einer höheren elektrischen Leitfähigkeit besteht, als ein Material aus dem der erste Abschnitt besteht, wobei der zweite Leitungsabschnitt auch für eine galvanische Verbindung mit einem Element außerhalb der Abflußleitung geeignet ist, wobei der zweite Abschnitt (36; 36'; 66) mindestens eine Innenfläche (41; 41'), die dafür vorgesehen ist, die Ablaufflüssigkeit zu berühren, und eine Außenfläche, die geeignet ist, in elektrischem Kontakt mit einem Stützelement (44; 69) verbunden zu werden, aufweist, wobei sich das Stützelement (44; 69) außerhalb der Abflussleitung befindet, wobei der zweite Abschnitt ein Anschlussstück (36; 36'; 66) umfasst, das einen röhrenförmigen Körper (37; 37') umfasst, der an zwei entgegengesetzten Enden davon zwei Verbindungsbereiche (38, 39; 38', 39') aufweist, wobei der röhrenförmige Körper (37; 37') aus einem elektrisch leitfähigen Material besteht; wobei der erste Abschnitt zwei röhrenförmige Elemente (40; 40', 40") umfasst, wobei jedes davon einen Endbereich besitzt, der mit einem der zwei Verbindungsbereiche (38, 39; 38'. 39') gekoppelt ist.

2. Kreislauf nach Anspruch 1, **dadurch gekennzeichnet, dass** die Abflussleitung (15b) ein Pumpsegment, das zur operativen Verbindung mit einer Pumpe (24) vorgesehen ist, zum Umlauf der Ablaufflüssigkeit entlang der Abflussleitung umfasst, und dass der elektrisch leitfähige zweite Abschnitt (36; 36') zwischen einem Eingangsende der Abflussleitung und dem Pumpsegment liegt, wobei das Eingangsende mit einem Ausgang der zweiten Kammer der Behandlungsvorrichtung (3) verbunden ist.

3. Kreislauf nach den Ansprüchen 1 oder 2, **dadurch gekennzeichnet, dass** der röhrenförmige Körper (37; 37') aus mindestens einem Kunststoffmaterial mit mindestens einem Zusatzstoff besteht, wobei der Zusatzstoff dem röhrenförmigen Körper (37; 37') elektrische Leitfähigkeitseigenschaften verleiht.

4. Kreislauf nach Anspruch 3, **dadurch gekennzeichnet, dass** der Zusatzstoff Kohlenstoff umfasst und das Kunststoffmaterial PVC umfasst.

5. Kreislauf nach einem der Ansprüche 1 bis 4, **dadurch gekennzeichnet, dass** das Anschlussstück eine Innenfläche (41; 41'), die dazu bestimmt ist, die Ablaufflüssigkeit zu berühren, und eine Außenfläche, die dazu bestimmt ist, ein außerhalb der Abflussleitung liegendes Element (44) elektrisch zu kontaktieren, aufweist, um elektrische Ladungen in der Ablaufflüssigkeit außen abzuleiten, und dass ein elektrischer Widerstand zwischen der Innenfläche und der Außenfläche zwischen 40 KΩund 10 MΩ beträgt.

6. Kreislauf nach Anspruch 5, **dadurch gekennzeichnet, dass** der elektrische Widerstand zwischen der Innenfläche und der Außenfläche zwischen 200 KΩ und 2 MΩ beträgt.

7. Kreislauf nach einem der Ansprüche 1 bis 6, **dadurch gekennzeichnet, dass** der röhrenförmige Körper (37') umfasst:
mindestens ein erstes axiales Anschlagelement (42; 43), das mit einem Endbereich eines ersten röhrenförmigen Elements (40'; 40") operativ verbunden ist, um eine axiale Einführung des ersten röhrenförmigen Elements (40'; 40") in den röhrenförmigen Körper zu begrenzen; und
mindestens ein zweites axiales Anschlagelement (42; 43), axial beabstandet vom ersten axialen Anschlagelement (42, 43) und operativ verbunden mit einem Endbereich eines zweiten röhrenförmigen Elements (40'; 40"), um eine axiale Einführung des zweiten röhrenförmigen Elements in den röhrenförmigen Körper (37') zu begrenzen; wobei die axiale Einführung des zweiten röhrenförmigen Elements (40'; 40") in den röhrenförmigen Körper (37') in einer der axialen Einführung des ersten röhrenförmigen Elements (40'; 40") entgegengesetzten Richtung erfolgt.

8. Kreislauf nach einem der Ansprüche 1 bis 7, **dadurch gekennzeichnet, dass**:
der röhrenförmige Körper (37, 37') einen freien Zwischenbereich aufweist, der nicht zur Verbindung verwendet wird und sich axial zwischen den Verbindungsbereichen (38, 39; 38', 39') befindet und zur Berührung mit der Ablaufflüssigkeit vorgesehen ist; und dass
der Zwischenbereich (41') einen Innendurchmesser aufweist, der kleiner ist als ein Innendurchmesser der Verbindungsbereiche (38', 39').

9. Kreislauf nach einem der Ansprüche 1 bis 8, **dadurch gekennzeichnet, dass** die Endbereiche der röhrenförmigen Elemente in den röhrenförmigen Körper eingeführt werden, wobei der röhrenförmige Körper einen axialen Zwischenbereich (41; 41') aufweist, der zwischen den Verbindungsbereichen (38, 39; 38', 39') liegt und von den Endbereichen der röhrenförmigen Elemente nicht verdeckt ist und in Berührung mit der Ablaufflüssigkeit ist.

10. Kreislauf nach einem der Ansprüche 1 bis 9, **dadurch gekennzeichnet, dass** der röhrenförmige Körper (37, 37') einstückig gefertigt ist.

11. Kreislauf nach einem der vorherigen Ansprüche, **dadurch gekennzeichnet, dass** das Material des ersten Leitungsabschnitts ein elastisch verformbares Kunststoffmaterial ist, und dass das Material des zweiten Leitungsabschnitts ein elastisch verformbares Kunststoffmaterial ist, mit dem Zusatz von mindestens einem Zusatzstoff mit Eigenschaften, die das Material des zweiten Leitungsabschnitts elektrisch leitfähig machen.

12. Kreislauf nach Anspruch 11, **dadurch gekennzeichnet, dass** der Zusatzstoff dem Material des zweiten Leitungsabschnitts eine höhere Steifigkeit verleiht.

13. Kreislauf nach einem der vorherigen Ansprüche, **dadurch gekennzeichnet, dass** er weitereine oder mehrere Leitungen zum Umlauf einer Flüssigkeit umfasst, die aus einer Gruppe der folgenden Leitungen ausgewählt ist:
mindestens eine Zuführungsleitung (15a) zur Zuführung einer operativen Flüssigkeit zur zweiten Kammer der Behandlungsvorrichtung;
mindestens eine erste Infusionsleitung (4) zur Infusion einer Ersatzflüssigkeit zur Blutentnahmeleitung (2a);
mindestens eine zweite Infusionsleitung (27) zur Infusion einer Ersatzflüssigkeit zur Blutrückgabeleitung (2b).

14. Vorrichtung zur extrakorporalen Blutbehandlung umfassend einen Kreislauf nach einem der Ansprüche 1 bis 13 und eine Maschine zur extrakorporalen Blutbehandlung zur Verwendung mit einem Kreislauf nach einem der Ansprüche 1 bis 13, wobei die Maschine umfasst:
mindestens eine Blutpumpe (9) zum Blutumlauf in der Blutentnahmeleitung (2a);
mindestens eine Abflusspumpe für Zirkulation der Ablaufflüssigkeit in der Abflussleitung (2b);
mindestens ein Stützelement (44), das geeignet ist, in elektrischem Kontakten elektrisch leitfähigen zweiten Leitungsabschnittaufzunehmen;
mindestens eine galvanische Verbindung (50, 51), die das Stützelement mit einer Außenmasse verbindet, um im Kreislauf gegebenenfalls vorhandene elektrische Ladungen abzuleiten.

15. Vorrichtung nach Anspruch 14, **dadurch gekennzeichnet, dass** die galvanische Verbindung eine Erdung umfasst.

16. Vorrichtung nach Anspruch 14 oder 15, **dadurch gekennzeichnet, dass** die galvanische Verbindung mindestens ein elektrisches Kabel (50) umfasst, das das Stützelement (44) mit einem ebenfalls geerdeten Maschinenkörper verbindet.

17. Vorrichtung nach einem der Ansprüche 14 bis 16, **dadurch gekennzeichnet, dass** sie mindestens einen elektrischenSicherheitswiderstand (51) mit einem vorgegebenen Wert umfasst, der entlang der galvanischen Verbindung angeordnet ist.

18. Vorrichtung nach Anspruch 17, **dadurch gekennzeichnet, dass** der Wert des elektrischen Widerstands (51) größer als 0,1 MΩ ist.

19. Vorrichtung nach Anspruch 17 oder 18, **dadurch gekennzeichnet, dass** der Wert des elektrischen Widerstands (51) gleich oder größer als 1,0 MΩist.

20. Vorrichtung nach einem der Ansprüche 17 bis 19, **dadurch gekennzeichnet, dass** sie eine Vielzahl von elektrischen Widerständen mit vorgegebenen Werten umfasst, die parallel entlang der galvanischen Verbindung angeordnet sind.

21. Vorrichtung nach einem der Ansprüche 14 bis 20, **dadurch gekennzeichnet, dass** das Stützelement (44) mindestens einen ersten Abschnitt (46) aus einem elektrisch leitfähigen Material, der an einer tragenden Struktur der Maschine fest angebracht ist und an dem die galvanische Verbindung (50) endet, und einen zweiten Abschnitt (49) aus einem Kunststoffmaterial, der mit mindestens einem Organ (49a) zur entfernbaren Befestigung des zweiten elektrisch leitfähigen Leitungsabschnitt daran ausgestattet ist, umfasst.

## Revendications

1. Circuit pour le traitement extracorporel de sang, comprenant:
un dispositif de traitement de sang (3) ayant un premier compartiment et un deuxième compartiment séparé dudit premier compartiment par une membrane semi-perméable;
au moins une conduite de prélèvement de sang (2a) apte à alimenter le sang enlevé d'un patient dans ledit premier compartiment;
au moins une conduite de retour de sang (2b) apte à restituer le sang traité au patient après que le sang sort dudit premier compartiment;
au moins une conduite d'évacuation (15b) apte à évacuer un fluide de décharge dudit deuxième compartiment;
**caractérisé en ce que** ladite conduite d'évacuation (15b) comprend au moins une première portion (40; 40', 40") et une deuxième portion (36; 36'; 66), les deux étant au moins partiellement destinées au contact avec le fluide de décharge, ladite deuxième portion (36; 36'; 66) étant réalisée dans un matériau ayant une conductivité électrique supérieure à celle d'un matériau constituant ladite première portion, ladite deuxième portion de conduite étant aussi prévu à une connexion galvanique à un élément extérieur à la conduite d'évacuation, ladite deuxième portion (36; 36'; 66) ayant au moins une surface intérieure (41; 41') destinée au contact avec le fluide de décharge, et au moins une surface extérieure apte à être associée en contact électrique avec un élément de support (44; 69), lequel élément de support (44; 69) est extérieur à la conduite d'évacuation, ladite deuxième portion comprenant un joint (36; 36'; 66) comprenant un corps tubulaire (37; 37') ayant à ses deux extrémités opposées deux zones de connexion (38, 39; 38', 39'), ledit corps tubulaire (37; 37') étant réalisé dans un matériau électriquement conducteur; ladite première portion comprenant deux éléments tubulaires (40; 40', 40"), chacun ayant une zone d'extrémité accouplée à une desdites deux zones de connexion (38, 39; 38', 39').

2. Circuit selon la revendication 1, **caractérisé en ce que** ladite conduit d'évacuation (15b) comprend un segment de pompe apte à être associé de façon opérationnelle avec une pompe (24) pour la circulation du fluide de décharge le long de ladite conduite d'évacuation, et **en ce que** ladite deuxième portion électriquement conductrice (36; 36') est placée entre une extrémité d'entrée de ladite conduite d'évacuation et ledit segment de pompe, ladite extrémité d'entrée étant reliée à une sortie dudit deuxième compartiment du dispositif de traitement (3).

3. Circuit selon les revendications 1 ou 2, **caractérisé en ce que** ledit corps tubulaire (37; 37') est réalisé dans au moins un matériau plastique contenant au moins un additif qui donne audit corps tubulaire (37; 37') des propriétés de conductivité électrique.

4. Circuit selon la revendication 3, **caractérisé en ce que** ledit additif comprend carbone et ledit matériau plastique comprend PVC.

5. Circuit selon une quelconque des revendications 1 à 4, **caractérisé en ce que** ledit joint a une surface intérieure (41; 41') apte à entrer en contact avec le fluide de décharge, et une surface extérieure apte à entrer en contact électrique avec un élément (44) extérieur à la conduite d'évacuation, afin de dissiper à l'extérieur des charges électriques présentes dans le fluide de décharge, et **en ce que** une résistance électrique entre ladite surface intérieure et ladite surface extérieure est comprise entre 40 KΩ et 10 MΩ.

6. Circuit selon la revendication 5, **caractérisé en ce que** ladite résistance électrique entre ladite surface intérieure et ladite surface extérieure est comprise entre 200 KΩ et 2 MΩ.

7. Circuit selon une quelconque des revendications 1 à 6, **caractérisé en ce que** ledit corps tubulaire (37') comprend:
au moins un premier élément d'arrêt axial (42; 43) associé de façon opérationnelle avec une zone d'extrémité d'un premier élément tubulaire (40'; 40") pour limiter une introduction axiale dudit premier élément tubulaire (40'; 40") dans ledit corps tubulaire; et
au moins un deuxième élément d'arrêt axial (42; 43) axialement espacé du premier élément d'arrêt axial (42, 43) et associé de façon opérationnelle avec une zone d'extrémité d'un deuxième élément tubulaire (40'; 40") pour limiter une introduction axiale dudit deuxième élément tubulaire dans ledit corps tubulaire (37'); l'introduction axiale dudit deuxième élément tubulaire (40'; 40") dans le corps tubulaire (37') étant réalisée dans une direction opposée à l'introduction axiale dudit premier élément tubulaire (40'; 40").

8. Circuit selon une quelconque des revendications 1 à 7, **caractérisé en ce que**:
ledit corps tubulaire (37, 37') a une zone intermédiaire libre non utilisée pour la connexion, laquelle zone est comprise axialement entre lesdites zones de connexion (38, 39; 38', 39') et apte à entrer en contact avec le fluide de décharge; et **en ce que**
ladite zone intermédiaire (41') a un diamètre intérieur inférieur à un diamètre intérieur desdites zones de connexion (38', 39').

9. Circuit selon une quelconque des revendications 1 à 6, **caractérisé en ce que** lesdites zones d'extrémité desdits éléments tubulaires sont introduites dans ledit corps tubulaire, lequel corps tubulaire présente une zone intermédiaire axiale (41; 41') comprise entre lesdites zones de connexion (38, 39; 38', 39'), qui n'est pas occupée par lesdites zones d'extrémité desdits éléments tubulaires et qui est en contact avec le fluide de décharge.

10. Circuit selon une quelconque des revendications 1 à 9, **caractérisé en ce que** ledit corps tubulaire (37; 37') est réalisé dans une seule pièce.

11. Circuit selon une quelconque des revendications précédentes, **caractérisé en ce que** le matériau de ladite première portion de conduite est un matériau plastique déformable élastiquement, et **en ce que** le matériau de ladite deuxième portion de conduite est un matériau plastique déformable élastiquement, avec l'addition d'au moins un additif ayant des propriétés grâce auxquelles le matériau de ladite deuxième portion de conduite devient électriquement conducteur.

12. Circuit selon la revendication 11, **caractérisé en ce que** ledit additif donne plus de rigidité au matériau de ladite deuxième portion de conduite.

13. Circuit selon une quelconque des revendications précédentes, **caractérisé en ce que** il comprend en outre une ou plusieurs conduites pour la circulation d'un fluide, sélectionnées dans un groupe de conduites comme dans la suite:
au moins une conduite d'alimentation (15a) apte à alimenter un fluide opérationnel dans ledit deuxième compartiment du dispositif de traitement;
au moins une première conduite d'infusion (4) pour l'infusion d'un fluide de substitution dans ladite conduite de prélèvement de sang (2a);
au moins une deuxième conduite d'infusion (27) pour l'infusion d'un fluide de substitution dans ladite conduite de retour de sang (2b).

14. Appareil pour le traitement extracorporel de sang comprenant un circuit selon une quelconque des revendications 1 à 13 et une machine pour le traitement extracorporel le sang apte à être utilisée avec un circuit selon une quelconque des revendications 1 à 13, la machine comprenant:
au moins une pompe de sang (9) pour la circulation du sang dans la conduite de prélèvement de sang (2a);
au moins une pompe d'évacuation (24) pour la circulation du fluide de décharge dans la conduite d'évacuation (2b);
au moins un élément de support (44) apte à recevoir en contact électrique ladite deuxième portion de conduite électriquement conductrice;
au moins une connexion galvanique (50, 51) reliant ledit élément de support à une masse extérieure, afin de dissiper des charges électriques éventuellement présentes dans ledit circuit.

15. Appareil selon la revendication 14, **caractérisé en ce que** ladite connexion galvanique comprend une mise à la masse.

16. Appareil selon la revendication 14 ou 15, **caractérisé en ce que** ladite connexion galvanique comprend au moins un câble électrique (50) reliant ledit élément de support (44) à une corps de machine à sa fois mis à la masse.

17. Appareil selon une quelconque des revendications 14 à 16, **caractérisé en ce que** il comprend au moins une impédance électrique de sécurité (51) ayant une valeur préétablie et rangée le long de ladite connexion galvanique.

18. Appareil selon la revendication 17, **caractérisé en ce que** la valeur de ladite impédance électrique (51) est supérieure à 0,1 MΩ.

19. Appareil selon la revendication 17 ou 18, **caractérisé en ce que** la valeur de ladite impédance électrique (51) est égale ou supérieure à 1,0 MΩ.

20. Appareil selon une quelconque des revendications 17 à 19, **caractérisé en ce que** il comprend une pluralité d'impédances électriques ayant des valeurs préétablies, rangées en parallèle le long de ladite connexion galvanique.

21. Appareil selon une quelconque des revendications 14 à 20, **caractérisé en ce que** ledit élément de support (44) comprend au moins une première portion (46) réalisée dans un matériau électriquement conducteur, fixée à une structure portante de la machine et en correspondance de laquelle ladite connexion galvanique (50) termine, et une deuxième portion (49) réalisée dans un matériau plastique, munie d'au moins un organe (49a) pour y fixer de façon amovible ladite deuxième portion de conduite électriquement conductrice.
